# EUROPÄISCHE PATENTANMELDUNG

(11) **EP 1 520 577 A1**
(43) Veröffentlichungstag der Anmeldung: **06.04.2005**
(21) Anmeldenummer: 04022767.0
(22) Anmeldetag: 24.09.2004
(51) Int. Cl.: A61K 7/48, A61K 35/78, A61P 17/10, A61P 17/06

(54) **Kosmetische oder dermatologische Zusammensetzung enthaltend Nachtkerzenöl**

(30) Priorität: 30.09.2003 DE 10347487
(71) Anmelder: Kneipp-Werke Kneipp-Mittel-Zentrale GmbH & CO. KG, 97082 Würzburg (DE)
(72) Erfinder: Mischek, Simone, 97204 Höchberg (DE); Landgraf, Sascha, 97332 Volkach (DE); Wohlfart, Rainer, 97230 Sulzfeld (DE)
(74) Vertreter: Dreiss, Fuhlendorf, Steimle & Becker

(57) **Zusammenfassung**

Die Erfindung betrifft eine kosmetische oder dermatologische Zusammensetzung zur topischen Verwendung enthaltend als primäre Wirkstoffe Nachtkerzenöl und unverseifbare Bestandteile eines weiteren pflanzlichen Öls in einem synergetischem Verhältnis sowie deren Verwendung zur Prophylaxe und Behandlung von Hauterkrankungen am Menschen.

## Beschreibung

Die Erfindung betrifft eine kosmetische oder dermatologische Zusammensetzung zur topischen Verwendung enthaltend als primäre natürliche Wirkstoffe Nachtkerzenöl und unverseifbare Bestandteile aus mindestens einem weiteren pflanzlichen Öl sowie deren Verwendung zur Prophylaxe und Behandlung von Hauterkrankungen sowie trockner /atopischer Haut des Menschen.

An einer modernen kosmetischen oder dermatologischen Zusammensetzung zur topischen Verwendung sind hohe Anforderungen zu stellen.

Die Haut übt als das größte Organ des Menschen zahlreiche lebenswichtige Funktionen aus. Sie schützt z.B. vor Kälte, Hitze, Strahlung, dem Einwirken chemischer Substanzen und Krankheitserregern. Sofern die Haut diese Barrierefunktion nicht mehr ausreichend erfüllt, können lokale Irritationen oder auch ganzkörperliche Beschwerden erfolgen.

Die Haut, insbesondere die Epidermis, ist als Barriereorgan des menschlichen Organismus in besonderem Maße äußeren Einwirkungen unterworfen. Diese Barrierefunktion wird unter anderem von Hautlipiden aufrechterhalten. Diese epidermalen Lipide wie z.B. Glycosphingolipide, Ceramide, Sterine und Sterinester, Fettsäuren, Triglyceride, n-Alkane oder verschiedenen polaren Lipiden werden im Keratinisierungsprozess freigesetzt.

In einem optimalen Zustand der Haut liegt ein ausgewogenes Verhältnis von Hautlipiden und Hautfeuchtigkeit vor. Durch dieses Gleichgewicht werden wichtige Eigenschaften der Haut, wie das Penetrationsvermögen, Wasserbindungsvermögen, Elastizität, Regenerationsfähigkeit oder die Widerstandsfähigkeit gegen Umwelteinflüsse und Noxen unterschiedlichster Art, bestimmt. Den Hautlipiden, besonders die Oberflächenlipiden, ist dabei eine übergeordnete Bedeutung zuzuschreiben.

Der äußere Lipidfilm der Haut stellt eine sehr komplex zusammengesetzte Emulsion aus verschiedenen Lipiden und dem Sekret der Schweißdrüsen, wie Harnstoff, Fettsäuren, anorganische Salze und Wasser, dar. Die Bestandteile der Ölphase stammen vorrangig aus den Absonderungen der Talgdrüsen, die u.a. Squalen, Cholesterol und Cholesterolester, Ester vom Wachstyp, Triglyceride und freie Fettsäuren enthalten.

Die Regulation des Wasser- und Feuchtigkeitsgehaltes ist eine der wichtigsten Funktionen der epidermalen Lipidmembran. Allerdings hat sie nicht nur eine Barrierewirkung gegen externe chemische und physikalische Einflüsse, sondern trägt auch zum Zusammenhalt der Hornschicht bei. Die Zusammensetzung dieser Lipide, die über die gesamte Hornschicht verteilt vorliegen, ist für die intakte Funktion der epidermalen Barriere und damit für die Wasserundurchlässigkeit der Haut von entscheidender Bedeutung.

Bei trockener oder atopisch veränderter Haut kommt es zu einer Verringerung dieser Hautlipide. Damit einhergehend kommt es zu einer Verringerung der Hautfeuchtigkeit und einer Erhöhung des transepidermalen Wasserverlustes.

Wie Untersuchungen in jüngster Zeit ergeben haben, handelt es sich bei den einzelnen Komponenten nicht nur um "einfache" Lipide, sondern es sind größtenteils echte Wirkstoffe, gekennzeichnet durch eine antimikrobielle Wirksamkeit. Eine stärkere keimtötende Wirkung war zwar für die freien Fettsäuren schon bekannt, konnte aber auch für die polaren Lipide und die Glycosphingolipide nachgewiesen werden (Testkeim: Staphylococcus aureus), die übrigen Bestandteile zeigten eine deutlich geringere Wirksamkeit (Miller S.J., Aly R., Shinefled H.R., Elias P.M.; in vitro and in vivo Antistaphylococceal Activity of Human Stratum Corneum Lipids, Arch Dermato. 124, 209-215 (1983)).

Die in und auf der menschlichen Haut ebenfalls enthaltenen Phytosterine stammen aus der täglichen Nahrungsaufnahme. Innerhalb der Gruppe der Hautlipide nehmen die Phytosterole insofern eine Sonderstellung ein, als sie ausschließlich exogenen (pflanzlichen) Ursprungs sind. Nach oraler Applikation benötigen sie einen Zeitraum von ca. 25 Tagen, bis sie den Lipidfilm erreichen. Dieser auffällige Verzögerungseffekt lässt sich dadurch erklären, dass sie im Bereich der Basalmembran in die Membranen der sich neu bildenden Epidermiszellen und/oder in deren Granula eingebaut werden.

Das atopische Ekzem (auch: atopische Dermatitis bzw. Neurodermitis) ist eine immer häufiger auftretende schubweise Hauterkrankung, die insbesondere Heranwachsende betrifft. Die Haut eines an Neurodermitis Erkrankten ist in der Akutphase hoch sensibel und kann allergisch auf psychische und physische Stressoren sowie eine ganze Reihe von individuell unterschiedlichen Umweltfaktoren und -noxen reagieren. In der Akutphase ist die Haut entzündet und der Betroffene leidet ganz besonders unter dem quälenden Juckreiz. Soweit bekannt, liegen der Neurodermitis keine Erkrankung der inneren Organe zugrunde, sondern entzündliche freie radikale produzierende Prozesse der äußeren Hautschichten, so dass durch externe Applikation von Salben, Cremes oder Gelen hier Einfluss genommen werden kann.

Unter Hautpflege ist in erster Linie zu verstehen, dass die natürliche Funktion der Haut als Barriere gegen Umwelteinflüsse (z.B. Schmutz, Chemikalien, Mikroorganismen) und gegen den Verlust von körpereigenen Stoffen gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen. Ziel der Hautpflege ist es ferner, den durch tägliches Waschen verursachten Fettund Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Erscheinungen der Hautalterung verzögern.

Aus WO 02/36092 ist bekannt, dass trocknende Öle samt penetrierenden Kohlenwasserstoffen zur Behandlung des atopischen Ekzems eingesetzt werden können. Nachteilig ist jedoch der Einsatz von organischen Lösungsmitteln und vorrangig synthetischer Inhaltsstoffe.

Ein Mittel zur Pflege der menschlichen Haut auf der Basis von gamma-Linolensäure in Kombination mit Nachtkerzenöl ist in der DE 43 10 015 A1 beschrieben.

In DE 198 00 982 A1 wird eine medizinische Salbenzubereitung beschrieben, die zur Behandlung von Neurodermitis, Schuppenflechte und von Ekzemen geeignet ist und als Bestandteil Nachtkerzenöl, Lavendelöl und D-Panthenol aufweist.

Der vorliegenden Erfindung liegt die Aufgabe zugrunde, eine kosmetische oder dermatologische Zusammensetzung zur topischen Verwendung bereitzustellen, die zur Pflege der Haut geeignet ist und die oben beschriebenen Anforderungen umfassend erfüllt.

Die Aufgabe wird überraschend und für den Fachmann nicht vorhersehbar erfüllt durch eine kosmetische oder dermatologische Zubereitung zur topischen Verwendung, die 0,1 - 50 Gew.-% Nachtkerzenöl sowie 0,1 - 15 Gew.-% unverseifbare Bestandteile aus mindestens einem weiteren pflanzlichen Öl enthält. In weiteren Ausführungsformen werden vorzugsweise 1 - 30 Gew.-%, besonders bevorzugt 2 - 20 Gew.-% Nachtkerzenöl sowie vorzugsweise 0,1 - 10 Gew.-%, besonders bevorzugt 1 - 10 Gew.-% unverseifbare Bestandteile aus mindestens einem weiteren pflanzlichen Öl in der erfindungsgemäßen Zubereitung eingesetzt. Zudem stehen Nachtkerzenöl und unverseifbare Bestandteile aus mindestens einem weiteren pflanzlichen Öl in einem systemischen Zusammenhang unter Ausnutzung synergetischer Effekte im Rahmen der angegeben Gew.-%. So können die angegebenen Mengen an Nachtkerzenöl und unverseifbaren Bestandteilen aus mindestens einem weiteren pflanzlichen Öl als primäre Wirkstoffe zur üblichen Pflege der Haut als auch gleichzeitig zur Behandlung von Hauterkrankungen und trockner / atopischer Haut effizient und komplex verwendet werden und beste Ergebnisse erzielen.
Im Rahmen dieser Erfindung wird unter Nachtkerzenöl, das aus dem Samen der Nachtkerze (Oenothera biennis L.) durch Pressung / Extraktion und anschließender Raffination (Entsäuern, Bleichen, Dämpfen) erhaltene Öl verstanden. Das Öl ist von hellgelber Farbe und in Geruch und Geschmack fast neutral. Das Öl ist reich an einfach- und mehrfach ungesättigten Fettsäuren insbesondere an den beiden essentiellen Fettsäuren Linolensäure und gamma - Linolensäure.
In einer bevorzugten Ausführungsform ist daher das Nachtkerzenöl wie folgt charakterisiert:

| | |
|---|---|
| Dichte (20°C) | 0,920 -0,930 |
| Brechungsindex (20°C) | 1,475 -1,480 |
| Säurezahl | max. 0,6 |
| Peroxidzahl | max. 10 |
| Jodzahl | 145- 162 |
| Verseifungszahl | 184- 194 |
| Unverseifbare Anteile | max. 2,5 Gew. -% |

Enthaltend eine Fettsäurezusammensetzung, vorzugsweise von (in Gew. %):

| | | |
|---|---|---|
| Palmitinsäure | C 16:0 | 4,0 -8,0 % |
| Stearinsäure | C 18:0 | 0,5 -3,0 % |
| Ölsäure | C 18:1 | 5,0- 12,0 % |
| Linolsäure | C 18:2 | 66,0 -76,0 % |
| Linolensäure α | C 18:3 | max. 1,0 % |
| Linolensäure γ (kurz: GLA) | C 18:3 | 8,0- 12,0 % |
| Arachinsäure | C 20:0 | max. 1,5 % |
| Behensäure | C 22:0 | max. 2,0 % |
| Erucasäure | C 22:1 | nur in Spuren |
| Sonstige | | max. 2,0 % |

Nachtkerzenöl ist käuflich erhältlich (z.B. Lamotte, Bremen, Germany).

Im Rahmen dieser Erfindung wird unter "unverseifbare Bestandteile aus mindestens einem weiteren pflanzlichen Öl" die Gesamtheit der fettlöslichen und wasserunlöslichen organischen Bestandteile aus mindestens einer Pflanze verstanden, die durch eine Verseifungsreaktion nicht in Salze überführt werden können (z.B. nach DIN 53 900, technische Vorschrift, Germany) und zudem nicht im Nachtkerzenöl enthalten sind. Pflanzliche Fette enthalten zumeist 0,1 - 5 Gew.-% unverseifbare Bestandteile. Die Zusammensetzung dieser unverseifbaren Bestandteile können nicht abschließend sein: aromatische Verbindungen, Terpene, Tocopherole, Carotine, C-16- bis C-22-Kohlenwasserstoffe, Terpenalkohole, C-16 bis C-22-Alkohole sowie verschiedene Sterine, z.B. Cholesterin, β-Sitosterin, Stigmasterin, Campesterin, Brassicasterin.

Ganz besonders bevorzugt sind jedoch erfindungsgemäß unverseifbare Extrakte aus Pflanzenöle enthaltend Phytosterine (auch: Phytosterole) mit mindestens 25 Gew.-% an Phytosterinen. Phytosterine können in Pflanzen aus Sprossen, Keimen, Samen und Früchten erhalten werden. Erfindungsgemäß bevorzugte Phytosterine sind Stigmasterin und β-Sitosterin und pflanzliche Extrakte mit hohen Anteile an Stigmasterin und β-Sitosterin im Pflanzenöl.

Weiterhin bevorzugt im Sinne der Erfindung sind insbesondere Zusammensetzungen, die als unverseifbaren Bestandteil eines weiteren von Nachtkerzenöl verschiedenes Öl, nämlich die unverseifbaren Bestandteile aus der Familie Lauracea, insbesondere der Nutzpflanze Persea (z.B. persea americana, persea gratissima (Avocado)) umfassen (nachstehend auch Avocado-Extrakt). Besonders bevorzugt sind solche unverseifbaren Bestandteile der Persea, die 25 - 30 Gew. -% Phytosterine enthalten. Diese Phytosterine setzen sich hauptsächlich vorteilhaft aus β-Sitosterol und Stigmasterol neben anderen wie z.B. Campesterol und Brassicasterol zusammen. Ein besonders bevorzugter Avocado-Extrakt enthaltend unverseifbare Anteile-des Avocadoöles ist das käuflich erhältliche Avocadin ® (Croda, Nettetal, Germany).

Durch ihre scheibchenförmige Molekülstruktur begünstigten die Phytosterine eine Filmbildung auf der Haut, erschweren auf diese Weise die Resorption von toxischen Substanzen und sorgen für eine gute Hautfeuchtigkeit durch Verhinderungen eines übermäßigen transepidermalen Wasserverlustes. Des weiteren ergänzen sie systemisch die Wirkung des Nachtkerzenöls in der erfindungsgemäßen Zubereitung.

In einer weiteren Ausführungsform kann die erfindungsgemäße kosmetischen oder dermatologischen Zubereitung zur topischen Verwendung in Form einer Creme, Lotion (Hautmilch), Paste, Hautöl oder Emulsion erfolgen. Ebenfalls sind wasserfreie Systeme möglich.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert. Sind die beiden Flüssigkeiten Wasser und Öl und liegen Öltröpfchen fein verteilt in Wasser vor, so handelt es sich um eine Öl-in-Wasser-Emulsion (O/W-Emulsion). Der Grundcharakter einer O/W-Emulsion ist durch das Wasser geprägt. Bei einer Wasser-in-Öl-Emulsion (W/O-Emulsion) handelt es sich um das umgekehrte Prinzip, in dem der Grundcharakter hier durch das Öl bestimmt wird. Weiterhin sind Mischsysteme wie Wasser-in-Öl-in-Wasser-Emulsionen (W/O/W-Emulsion) und Ölin-Wasser-in-Öl-Emulsionen (O/W/O-Emulsionen) bekannt. Alle genannten Emulsionen sind erfindungsgemäß geeignet.

Zu den erfindungsgemäß geeigneten wasserfreien Systemen gehören reine Ölzubereitungen wie z.B. Hautöle. Ebenfalls einsetzbare Pasten enthaltend die erfindungsgemäße Zubereitung, zeichnen sich dadurch aus, das sie aus die gleichen oder ähnlichen Bestandteilen wie eine Emulsion besteht aber im wesentlichen wasserfrei sind. Im Rahmen der vorliegenden Erfindung werden die Begriffe Ölphase und Lipidphase synonym verwendet. In einer weiteren bevorzugten Ausführungsform kann die erfindungsgemäße Zubereitung als weiteren Bestandteil einen Emulgator beinhalten. In einer ganz bevorzugten Ausführung kann dieser Emulgator ein O/W-Emulgator sein.

Emulgatoren können vorteilhaft gewählt werden aus der Gruppe der nichtionischen, anionischen, kationischen oder amphoteren Emulgatoren.

Als nichtionischen Emulgator können verschiedene Emulgatoren aus den Gruppen der Partialfettsäureester, Fettalkohole, Sterole, Polyethylengylcole wie z.B. ethoxylierte Fettsäuren, ethoxylierte Fettalkohole und ethoxylierte Sorbitanester, Zuckeremulgatoren, Polyglycerinemulgatoren oder Silikonemulgatoren Verwendung finden.

Als anionischen Emulgator können verschiedene Emulgatoren aus den Gruppen der Seifen z. B. Natriumstearat, Fettalkoholsulfate, Mono-, Di- und Trialkylphosphosäureester und deren Ethoxylate, Fettsäure Lactat Ester, Fettsäure Citrat Ester oder Fettsäure Citroglycerinester Verwendung finden.

Als kationischen Emulgatoren können beispielsweise. quaternäre Ammoniumverbindungen mit einem langkettigen aliphatischen Rest z. B. Distearyldimonium Chloride eingesetzt werden.

Unter den amphoteren Emulgatoren können verschiedene Emulgatoren aus den Gruppen Alkylamininoalkancarbonsäuren, Betaine, Sulfobetaine oder Imidazolinderivate Verwendung finden.

Erfindungsgemäß bevorzugt sind natürlich vorkommende Emulgatoren, zu denen beispielsweise Bienenwachs, Wollwachs, Lecithin und Sterole u.a. gehören die ebenfalls bei der Herstellung einer erfindungsgemäßen Zubereitung eingesetzt werden können. In einer bevorzugten Formulierung der erfindungsgemäßen Zubereitung können O/W-Emulgatoren ausgewählt werden aus der Gruppe der Pflanzenproteinhydrolysaten und deren Derivate.

Zur Herstellung der erfindungsgemäßen Zubereitung lassen sich in vorteilhafter Weise die Ester und Salze von Weizenproteinhydrolysaten sowie Mono und Diester von Partialglyceriden als O/W-Emulgatoren verwenden. Ganz besonders bevorzugt werden Mischungen aus dem Weizenproteinhydrolysat (Potassium Palmitoyl Hydrolyzed Wheat Protein), Glycerylstearat und Cetearylalkohol eingesetzt. Insbesondere eignet sich eine Mischung bestehend aus 10 - 25 Gew.-% Potassium Palmitoyl Hydrolyzed Wheat Protein, 25 - 50 Gew.-% Glycerylstearat und 25 - 50 Gew. -% Cetearylalkohol zur Herstellung der erfindungsgemäßen Zubereitung. Eine derartige Kombination ist unter dem Handelsnamen Phytocream erhältlich.

Im Sinne der vorliegenden Erfindung können ferner vorteilhaft Substanzen als Zusatzstoffe enthalten sein, ausgewählt aus der Gruppe der Ester aus gesättigten und/oder ungesättigten, verzweigten und/oder unverzweigten Alkancarbonsäuren und/oder Alkencarbonsäuren mit einer Kettenlange von 3 - 30 Kohlenstoffatomen und gesättigten und/oder ungesättigten, verzweigten und/oder unverzweigten Alkoholen einer Kettenlange von 3 - 30 Kohlenstoffatomen sowie aus der Gruppe der Ester aus aromatischen Carbonsäuren und gesättigten und/oder ungesättigten, verzweigten und/oder unverzweigten Alkoholen einer Kettenlange von 3 bis 30 C-Atomen. Solche Esterole konnen dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecyl-palmitat, Oleyloleat, Oleylerucat, Erucyloleat, Erucylerucat sowie synthetische, halbsynthetische und natürliche Gemische solcher Ester, wie z.B. Jojobaöl.

Jojobaöl wird aus den reifen Samen des Jojobastrauches (*Simmondsia chinensis*) durch mechanisches Pressung und anschließender Raffination gewonnen. Das Öl ist eine klare wasserhelle Flüssigkeit, die fast keinen Geruch aufweist. Das Jojobaöl gleicht in seiner chemischen Struktur einem Wachs, das einer langkettigen einfach ungesättigten Fettsäure aufgebaut ist, insbesondere aus Gadoleinsäure und Erucasäure.

Ferner kann die Ölphase vorteilhaft gewählt werden aus der Gruppe der verzweigten und unverzweigten. Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 - 24 C-Atomen, insbesondere 12 - 18 C-Atomen. Die Fettsäuretriglyceride können beispielsweise vorteilhaft gewählt werden aus der Gruppe der synthetischen, halbsynthetischen und natürlichen Öle.

Ebenfalls geeignet ist Erdnussöl, welches aus dem Samen der subtropischen Erdnusspflanze (*Arachis Hypogaea*) durch Pressung und Extraktion gewonnen. Anschließend erfolgt die Entschleimung (degumming), Filtration und Raffination (Entsäuern, Bleichen, Dämpfen). Man erhält dadurch ein blass-gelbliches Öl mit leicht an die Samen erinnerndem Geruch und Geschmack. Das Öl ist reich an einfach- und mehrfach-ungesättigten Fettsäuren insbesondere an den beiden essentiellen Fettsäuren Ölsäure und Linolsäure.

Weiterhin geeignet ist Mandelöl, ein kaltgepresstes, raffiniertes, fettes Öl aus den reifen Samen von *Prunus dulcis* (Miller) D.A. Webb var. *dulcis* oder *Prunus dulcis* (Miller) D.A. Webb var. *amara* (D.C.) Buchheim oder aus einer Mischung von beiden. Das Öl ist eine hellgelbe, klare, durchscheinende Flüssigkeit mit schwachem, charakteristischem Geruch und schwachem charakteristischem, süßlichem Geschmack. Das Mandelöl ist reich an einfach- und mehrfach ungesättigten Fettsäuren insbesondere an den beiden essentiellen Fettsäuren Ölsäure und Linolsäure.

Beispiele geeigneter Fettsäurezusammensetzungen einiger pflanzlicher Öle und Wachse als Zusatzstoffe

| Fettsäure | | Mandelöl | Erdnussöl | Jojobaöl | Nachtkerzenöl |
|---|---|---|---|---|---|
| | < C 16:0 | 0,0 - 0,1 % | 0,0 - 0,4 % | | |
| Palmitinsäure | C 16:0 | 4,0 - 9,0 % | 7,0 - 16,0 % | 0,0 - 3,0 % | 4,0 - 8,0 % |
| Stearinsäure | C 18:0 | 0,0 - 3,0 % | 1,3 - 6,5 % | | 0,5 - 3,0 % |
| Ölsäure | C 18:1 | 62,0 - 86,0 % | 35,0 - 72,0 % | 5,0 - 15,0 % | 5,0 - 12,0 % |
| Linolsäure | C 18:2 | 7,0 - 30,0 % | 13,0 - 43,0 % | | 66,0 - 76,0.% |
| α-Linolensäure | C 18:3 | 0,0 - 0,2 % | 0,0 - 0,6 % | | 0,0 - 1,0 % |
| γ -Linolensäure | C 18:3 | 0,0 - 0,2 % | 0,0 - 0,6 % | | 8,0 - 12,0 % |
| Arachinsäure | C 20:0 | 0,0 - 0,1 % | 0,5 - 3,0 % | | 0,0 - 1,5 % |
| Gadolinäure | C 20:1 | 0,0 - 0,1 % | 0,5 - 2,1 % | 65,0 - 80,0 % | |
| Behensäure | C 22:0 | 0,0 - 0,1 % | 1,0 - 5,0 % | 0,0 - 1,0 % | 0,0 - 2,0 % |
| Erucasäure | C 22:1 | 0,0 - 0,1 % | 0,0 - 0,5 % | 10,0 - 20,0 % | 0,0 - 0,5 % |
| Lignocerinsäure | C 24:0 | | 0,5 - 3,0 % | | |
| Nervonsäure | C 24:1 | | | 0.0.-3,0% | |
| sonstige | | | | Max. 3% | max. 2,0 % |

Daher betrifft die Erfindung ebenfalls eine erfindungsgemäße Zubereitung, die als Zusatzstoffe, beispielsweise Jojobaöl, Olivenöl, Sonnenblumenöl, Sojaöl, Erdnussöl, Rapsöl, Mandelöl, Palmöl, Kokosöl, Palmkernöl oder dergleichen enthält. Ganz besonders bevorzugt sind pflanzliche Öle, die mindestens 6 Gew. -% an mehrfach ungesättigten Fettsäuren aufweisen.

In einer weiteren bevorzugten Ausführungsform enthalten die erfindungsgemäßen Zubereitungen mindestens 1 Gew. -%, vorzugsweise 1 - 30 Gew. -%, besonders bevorzugt 2 - 15 Gew. -% oder gar 5-10 Gew. -% eines solchen, mindestens einem weiteren pflanzlichen Öls als Zusatzstoff.

Insbesondere können in den erfindungsgemäßen Zubereitungen zusätzlich als Hilfs- oder Zusatzstoff Antioxidantien und/oder Radikalfängern zugesetzt werden. Vorteilhaft werden solche Antioxidantien gewählt aus der Gruppe der lipophilen System beispielsweise: natürliche und synthetische Tocopherole, Nordihydroguajaretsäure, Coniferylbenzoat, Butylhydroxyanisol, Butlyhydroxy-tolul, Gallussäureester und verschiedenen antioxidative Pflanzen Extrakte. Unter den hydrophilen Systemen sind besonders vorteilhaft anorganischen Schwefelverbindungen, Natriumhydrogensulfit, Cystein oder Ascorbinsäure zu verwenden.

Vorteilhaft lassen sich die erfindungsgemäßen Zusammensetzungen mit einem Zusatz von antioxidativen Pflanzenextrakten stabilisieren. Besonders vorteilhaft sind Rosmarinextrakte und zwar 0,001 - 10 Gew.-%, vorzugsweise 0,01 - 5 Gew. -% in der erfindungsgemäßen Zubereitung enthaltend. Vorzugsweise solche, die mit Hilfe eines mit überkritischen Kohlendioxid (CO₂) durchgeführten Extraktionsverfahren gewonnen werden können (beispielsweise beschrieben in EP 0454 097 B1). Erfindungsgemäß besonders geeignet sind zudem Rosmarinextrakte mit einer Carnolsäurekonzentration von mindestens 15 Gew.-%.

Rosmarinextrakte zeigen zudem eine vorteilhafte systemische Unterstützung der erfindungsgemäßen Zubereitung in ihrer komplexen Wirkung.

Die erfindungsgemäßen kosmetischen und dermatologischen Zubereitungen können weiterhin kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, weichmachende, anfeuchtende und/oder feuchthaltende Substanzen oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

In einer weiteren besonderen Ausführungsform ist die erfindungsgemäße Zubereitung im wesentlichen aus natürlich vorkommenden Inhaltsstoffen zusammengesetzt, wie oben im einzelnen genannt.

Des weiteren betrifft die Erfindung die Verwendung der erfindungsgemäßen Zusammensetzung zur Prophylaxe trockener oder atopischer Haut und Hautalterungserscheinungen, insbesondere Faltenbildung sowie zur Prophylaxe von Hauterkrankungen des Menschen, insbesondere atopisches Ekzem bzw. Neurodermitis, Akne und Psoriasis bzw. Schuppenflechte.

Zudem betrifft die Erfindung ein Arzneimittel zur Behandlung von Hauterkrankungen am Menschen, insbesondere atopisches Ekzem bzw. Neurodermitis, Akne und Psoriasis bzw. Schuppenflechte. Das Arzneimittel kann zu diesen Zwecken äußerlich auf die Haut des Menschen aufgetragen werden.

Nachstehende Beispiele dienen zur näheren Erläuterung der Erfindung, ohne die Erfindung auf diese Beispiele zu beschränken.

Die INCI-Nomenklatur kann zu den vor- und nachstehenden Stoffen entsprechend verwendet werden.

### Beispiele:

### Beispiel 1:

Zusammensetzung einer Creme

| | Gew.-% |
|---|---|
| Wasser, | Ad 100 |
| demineralisiert | |
| Erdnussöl | 10,000 |
| Cetearylalkohol | 3,000 |
| Glycerylstearat | 3,000 |
| Potassium Palmitoyl | 2,000 |
| Hydrolyzed Wheat | |
| Protein | |
| Mandelöl | 6,000 |
| Jojobaöl | 6,000 |
| Avocado-Extrakt | 5,000 |
| Glycerin | 2,550 |
| Nachtkerzenöl | 2,500 |
| Dexpanthenol | 0,500 |
| Xanthan | 0,200 |
| Parfümöl | 0,200 |
| Tocopherol | 0,100 |
| Citronensäure | 0,039 |
| Rosmarinextrakt | 0,013 |

### Beispiel 2:

Zusammensetzung einer Lotion (Hautmilch)

| | Gew.-% |
|---|---|
| Wasser, | Ad 100 |
| demineralisiert | |
| Erdnussöl | 10,000 |
| Mandelöl | 6,000 |
| Jojobaöl | 6,000 |
| Potassium Palmitoyl | 1,125 |
| Hydrolyzed Wheat | |
| Protein | |
| Cetearylalkohol | 1,688 |
| Glycerylstearat | 1,688 |
| Avocado-Extrakt | 4,000 |
| Nachtkerzenöl | 3,500 |
| Glycerol | 2,550 |
| Dexpanthenol | 0,500 |
| Tocopherol | 0,200 |
| Xanthan | 0,200 |
| Parfümöl | 0,100 |
| Citronensäure | 0,039 |
| Rosmarinextrakt | 0,013 |

## Patentansprüche

1. Kosmetische oder dermatologische Zusammensetzung zur topischen Verwendung, enthaltend 0,1 - 50 Gew.-% Nachtkerzenöl und 0,1 - 15 Gew.-% unverseifbare Bestandteile aus mindestens einem weiteren pflanzlichen Öl und weitere Hilfs- und/oder Zusatzstoffe.

2. Kosmetische oder dermatologische Zusammensetzung zur topischen Verwendung nach Anspruch 1, **dadurch gekennzeichnet, dass** als primäre Wirkstoffe 0,1 - 50 Gew.-% Nachtkerzenöl und 0,1 - 15 Gew.-% unverseifbare Bestandteile aus mindestens einem weiteren pflanzlichen Öl enthalten ist.

3. Kosmetische oder dermatologische Zusammensetzung zur topischen Verwendung nach Anspruch 1 oder 2, in Form einer Creme, Lotion, Paste, Hautöl oder Emulsion.

4. Kosmetische oder dermatologische Zusammensetzung zur topischen Verwendung nach einem der Ansprüche 1 bis 3, **dadurch gekennzeichnet dass**, die unverseifbaren Bestandteile eines pflanzlichen Öls aus einem Extrakt der Nutzpflanze Persea stammt.

5. Kosmetische oder dermatologische Zusammensetzung zur topischen Verwendung nach einem der Ansprüche 1 bis 4, **dadurch gekennzeichnet dass**, die unverseifbaren Bestandteile des weiteren pflanzlichen Öls mindestens 25 Gew.-% an Phytosterinen enthalten.

6. Kosmetische oder dermatologische Zusammensetzung zur topischen Verwendung nach einem der Ansprüche 1 bis 5, **dadurch gekennzeichnet, dass** als Zusatzstoff mindestens ein weiteres pflanzliches Öl enthalten ist, das mindestens 6 Gew. -% an mehrfach ungesättigten Fettsäuren enthält.

7. Kosmetische oder dermatologische Zusammensetzung zur topischen Verwendung nach einem der Ansprüche 1 bis 6, **dadurch gekennzeichnet, dass** ein oder mehrere Zusatzstoffe ausgewählt sind aus der Gruppe Jojobaöl, Olivenöl, Sonnenblumenöl, Sojaöl, Erdnussöl, Rapsöl, Mandelöl, Palmöl, Kokosöl, Palmkernöl.

8. Kosmetische oder dermatologische Zusammensetzung zur topischen Verwendung nach Anspruch 6 oder 7, **dadurch gekennzeichnet, dass** das weitere pflanzliche Öl zu mindestens 1 Gew. -%, 1 - 30 Gew. -% oder vorzugsweise 2 - 15 Gew.-% in der Zubereitung enthalten ist.

9. Kosmetische oder dermatologische Zusammensetzung zur topischen Verwendung nach einem der Ansprüche 1 bis 8, **dadurch gekennzeichnet, dass** als Zusatzstoff 0,001 - 10 Gew.-% Rosmarinextrakt enthalten ist.

10. Kosmetische oder dermatologische Zusammensetzung zur topischen Verwendung nach Anspruch 9, wobei das Rosmarinextrakt mittels Kohlendioxidextraktion gewonnen wird.

11. Kosmetische oder dermatologische Zusammensetzung zur topischen Verwendung nach Anspruch 9 oder 10, wobei das Rosmarinextrakt mindestens 15 Gew.-% Carnosolsäure enthält.

12. Kosmetische oder dermatologische Zusammensetzung zur topischen Verwendung nach einem der Ansprüche 1 bis 11, **dadurch gekennzeichnet, dass** ein O/W-Emulgator, insbesondere ein Pflanzenproteinhydrolysat, enthalten ist.

13. Kosmetische oder dermatologische Zusammensetzung zur topischen Verwendung nach einem der Ansprüche 1 bis 12, bestehend aus
| | Gew.-% |
|---|---|
| Wasser, | Ad 100 |
| demineralisiert | |
| Erdnussöl | 10,000 |
| Cetearylalkohol | 3,000 |
| Glycerylstearat | 3,000 |
| Potassium Palmitoyl Hydrolyzed Wheat Protein | 2,000 |
| Mandelöl | 6,000 |
| Jojobaöl | 6,000 |
| Avocado-Extrakt | 5,000 |
| Glycerin | 2,550 |
| Nachtkerzenöl | 2,500 |
| Dexpanthenol | 0,500 |
| Xanthan | 0,200 |
| Parfümöl | 0,200 |
| Tocopherol | 0,100 |
| Citronensäure | 0,039 |
| Rosmarinextrakt | 0,013 |
in Form einer Creme.

14. Kosmetische oder dermatologische Zusammensetzung zur topischen Verwendung nach einem der Ansprüche 1 bis 12, bestehend aus
| | Gew.-% |
|---|---|
| Wasser, demineralisiert | Ad 100 |
| Erdnussöl | 10,000 |
| Mandelöl | 6,000 |
| Jojobaöl | 6,000 |
| Potassium Palmitoyl Hydrolyzed Wheat Protein | 1,125 |
| Cetearylalkohol | 1,688 |
| Glycerylstearat | 1,688 |
| Avocado-Extrakt | 4,000 |
| Nachtkerzenöl | 3,500 |
| Glycerol | 2,550 |
| Dexpanthenol | 0,500 |
| Tocopherol | 0,200 |
| Xanthan | 0,200 |
| Parfümöl | 0,100 |
| Citronensäure | 0,039 |
| Rosmarinextrakt | 0,013 |
in Form einer Hautmilch.

15. Verwendung einer kosmetischen oder dermatologische Zusammensetzung nach einem der Ansprüche 1 bis 14 zur Prophylaxe trockener oder atopischer Haut und Hautalterungserscheinungen, insbesondere Faltenbildung.

16. Verwendung einer kosmetischen oder dermatologische Zusammensetzung nach einem der Ansprüche 1 bis 14 zur Prophylaxe von Hauterkrankungen des Menschen, insbesondere atopisches Ekzem bzw. Neurodermitis, Akne und Psoriasis bzw. Schuppenflechte.

17. Verwendung einer dermatologischen Zusammensetzung nach einem der Ansprüche 1 bis 14 zur Behandlung von Hauterkrankungen des Menschen, insbesondere atopisches Ekzem bzw. Neurodermitis, Akne und Psoriasis bzw. Schuppenflechte.

18. Arzneimittel enthaltend eine dermatologische Zusammensetzung nach einem der Ansprüche 1 bis 14 zur Behandlung von Hauterkrankungen am Menschen, insbesondere atopisches Ekzem bzw. Neurodermitis, Akne und Psoriasis bzw. Schuppenflechte.
